(19) **Europäisches Patentamt European Patent Office Office européen des brevets**

(11) **EP 3 328 923 B1**

(12) **FASCICULE DE BREVET EUROPEEN**

(45) Date de publication et mention
de la délivrance du brevet:
**11.03.2026 Bulletin 2026/11**

(21) Numéro de dépôt: **16770035.0**

(22) Date de dépôt: **27.07.2016**

(51) Classification Internationale des Brevets (IPC):
**C08J 9/00** *(2006.01)*    **A61L 27/20** *(2006.01)*
**A61L 27/56** *(2006.01)*    **A61K 47/36** *(2006.01)*
**C08J 9/08** *(2006.01)*    **C08J 9/28** *(2006.01)*

(52) Classification Coopérative des Brevets (CPC):
(C-Sets disponibles)
**C08J 9/28; A61L 27/20; A61L 27/26; A61L 27/56;**
**A61L 27/58; C08J 9/0061; C08J 9/08;**
A61L 2430/20; C08J 2201/026; C08J 2201/0546;
C08J 2203/02; C08J 2205/026; C08J 2205/044;
C08J 2205/05; C08J 2207/10;                (Cont.)

(86) Numéro de dépôt international:
**PCT/FR2016/051954**

(87) Numéro de publication internationale:
**WO 2017/017379 (02.02.2017 Gazette 2017/05)**

(54) **PROCÉDÉ DE PRÉPARATION DE MATRICES POREUSE POLYMÈRES BIOCOMPATIBLES ET BIODEGRADABLES TRIMENSIONNELLES**

VERFAHREN ZUR HERSTELLUNG VON BIOKOMPATIBLEN UND BIOLOGISCH ABBAUBAREN PORÖSEN DREIDIMENSIONALEN POLYMERMATRIZEN

PROCESS FOR PREPARING BIOCOMPATIBLE AND BIODEGRADABLE POROUS THREE-DIMENSIONAL POLYMER MATRICES

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité:  **30.07.2015  FR 1557339**

(43) Date de publication de la demande:
**06.06.2018  Bulletin 2018/23**

(72) Inventeurs:
- **GIROD FULLANA, Sophie**
  **31320 Auzeville Tolosane (FR)**
- **SALLERIN, Brigitte**
  **31400 Toulouse (FR)**
- **BUSHKALOVA, Raya**
  **31400 Toulouse (FR)**
- **CECCALDI, Caroline**
  **82140 Saint Antonin Noble Val (FR)**

(73) Titulaires:
- **Université Paul Sabatier Toulouse III**
  **31062 Toulouse (FR)**
- **Centre National de la Recherche Scientifique**
  **75016 Paris (FR)**
- **INSERM (Institut National de la Santé et de la Recherche Scientifique)**
  **75013 Paris (FR)**
- **Centre Hospitalier Universitaire de Toulouse**
  **31059 Toulouse (FR)**

(74) Mandataire: **Ipsilon**
**12 Avenue d'Italie**
**75013 Paris (FR)**

(56) Documents cités:
**WO-A1-2008/092228     WO-A2-2005/018492**

• CHHAVI SHARMA ET AL: "Fabrication and characterization of natural origin chitosan-gelatin-alginate composite scaffold by foaming method without using surfactant", JOURNAL OF APPLIED POLYMER SCIENCE, vol. 127, no. 4, 19 May 2012 (2012-05-19), US, pages 3228 - 3241, XP055280298, ISSN: 0021-8995, DOI: 10.1002/app.37755
• JUN YANG ET AL: "Hepatocyte-specific porous polymer-scaffolds of alginate/galactosylated chitosan sponge for liver-tissue engineering", BIOTECHNOLOGY LETTERS, 1 September 2001 (2001-09-01), Dordrecht, pages 1385 - 1389, XP055280289, Retrieved from the Internet <URL:http://rd.springer.com/content/pdf/10.1023/A:1011600414225.pdf> DOI: 10.1023/A:1011600414225

(52) Classification Coopérative des Brevets (CPC):
(Cont.)C08J 2305/04; C08J 2305/08; C08J 2405/04; C08J 2405/08

C-Sets
A61L 27/20, C08L 5/04;
A61L 27/20, C08L 5/08;
A61L 27/26, C08L 5/04;
A61L 27/26, C08L 5/08

**Description**

La présente invention est relative à un procédé de préparation d'une matrice polymère tridimensionnelle poreuse biocompatible et biodégradable, utilisable notamment comme support et pour la culture de cellules ou en médecine régénérative, en particulier pour la thérapie cellulaire cardiaque.

La thérapie cellulaire est une stratégie thérapeutique prometteuse des syndromes ischémiques cardiaques. En effet, ces derniers sont d'ores et déjà traités chez les patients par greffe autologue de cellules souches mésenchymateuses (CSM) directement injectées dans l'organe lésé, mais le faible taux de survie cellulaire (<15%) dans les trois jours suivant l'injection en limite les effets bénéfiques. Or, la récupération fonctionnelle de l'organe lésé est directement corrélée au nombre de CSM survivantes ; des études récentes ont montré que la régénération de l'organe infarci serait majoritairement due à leur effets paracrines. Dans ce contexte, maintenir la viabilité et de la fonctionnalité des CSM apparaît comme un enjeu majeur pour l'amélioration du rapport bénéfice/risque du traitement des ischémies par thérapie cellulaire.

L'élaboration de biomatériaux poreux, susceptibles de générer un environnement biomimétique favorable au maintien des CSM, tout en permettant une administration sous forme de *« patch »* à la surface de l'organe lésé, constitue une avancée dans ce domaine, et fait l'objet de recherches intensives.

Une telle approche, ne nécessitant plus l'injection des cellules directement dans le cœur, permettrait d'éviter: 1) la mortalité des cellules injectées, 2) les effets secondaires liés à l'injection intra-parenchymateuse (notamment les saignements) et 3) le danger de la transformation des cellules souches mésenchymateuses en un phénotype non souhaité (ostéoblaste, chondroblaste, adipocyte ou pire cellules cancéreuses). Pour cela, il est nécessaire de développer des matériaux poreux biomimétiques en 3 dimensions (3D) qui localisent les cellules sur le site lésé, les protègent lors de l'implantation et les maintiennent à un stade indifférencié pour prolonger leurs effets paracrines. Pour ce faire, les matériaux poreux tridimensionnels doivent répondre à un cahier des charges précis concernant leur porosité, leur résistance mécanique, et leur élasticité.

Les matrices polymères sont largement utilisées dans le secteur de l'ingénierie tissulaire. Généralement, on distingue deux grands types de matrices : celles faisant appel à des polymères d'origine synthétique et celles à base de polymères d'origine naturelle, ces dernières ayant la préférence en raison de leur meilleure biocompatibilité et de la possibilité de synthèse de matrices sans solvants organiques ou intermédiaires réactionnels toxiques, à des températures et des pH compatibles avec la survie cellulaire.

Au cours des 20 dernières années, l'alginate s'est révélé être le polymère de choix en ingénierie tissulaire en raison de son excellente biocompatibilité *in vivo,* attribuée à sa nature polysaccharidique, et au fait que la structure de son réseau s'apparente à celle de la matrice extracellulaire (MEC) des tissus vivants. Par exemple, Andersen T. et al. (BioMacromolecules, 2012, 13, 3703-3710) décrivent des éponges macroporeuses à pores ouverts et interconnectés à base d'alginate ainsi que leurs applications potentielles pour l'encapsulation de cellules, la délivrance de médicaments et la cicatrisation. Ces éponges macroporeuses sont préparées par gélification d'une solution d'alginate, de carbonate de calcium et d'un plastifiant en présence d'un cation divalent, ladite solution étant préalablement agitée à haute vitesse pour y incorporer des bulles d'air. Le gel obtenu est ensuite déshydraté par séchage à l'air à 80°C pour conduire à une structure hautement poreuse appelée xérogel. Ces xérogels d'alginate présentent un intérêt commercial limité pour toutes les applications nécessitant une implantation. En effet, ceux-ci présentent une porosité favorable à la culture de cellules mais qui, par contre, leur confère en contre-partie une faible résistance mécanique. De plus, il est difficile de maintenir leur structuration 3D initiale (responsable de leur biocompatibilité, de leur biomimétisme et de leur capacité d'ensemencement) après ce type de séchage.

La demande internationale WO 2007/103208 décrit un procédé de préparation d'éponges biodégradables à porosité ouverte à base d'un polysaccharide tel que le chitosan ou l'acide hyaluronique, consistant à former une mousse humide à partir d'une dispersion aqueuse comprenant un tel polysaccharide, un agent moussant et éventuellement un ou plusieurs ions formant un gel, un plastifiant, un agent de réticulation et un modificateur du pH, puis à mouler et sécher à l'air la mousse ainsi obtenue. Tout comme les éponges d'alginates précédentes, les éponges de polysaccharides ainsi obtenues (xérogels) présentent un réseau poreux ouvert mais des propriétés mécaniques insuffisantes pour une implantation aisée.

Le brevet US 6,425,918 décrit par ailleurs un procédé de préparation de matrices à base de polysaccharides tels que les alginates, les carraghénanes, la gomme gellane, la gomme xanthane, le chitosan, etc... consistant, dans une première étape, à gélifier une solution d'un polysaccharide en présence d'un agent réticulant, puis dans une deuxième étape à congeler le gel ainsi obtenu, avant de le sécher par lyophilisation dans une troisième étape. Les matrices obtenues selon ce procédé (cryogels) présentent une résistance mécanique améliorée par rapport aux xérogels d'alginate décrits dans Andersen T. *et al.* (pré-cité). Cependant, il est difficile par ce procédé de moduler la porosité générée par le mode de congélation et de séchage, et de l'adapter aux applications envisagées ; les possibilités de maîtrise des dimensions des pores obtenus sont limitées.

Il a également déjà été proposé, notamment dans l'article de Chhavi Sharma et al., Journal of Applied Polymer Science, 2012, Vol. 127(4), pages 3228-3241, une matrice composée d'une combinaison de 3 polymères alginate/-chitosan/gélatine. Cette matrice est préparée à partir d'une mousse obtenue par agitation d'une solution aqueuse d'alginate (2%) et de gélatine (5%) sans utiliser de tensio-actif mais en présence de $NaHCO_3$ pour générer du gaz, ladite mousse étant ensuite additionnée d'une solution de glutaraldéhyde. La mousse d'alginate/gélatine réticulée est ensuite ajoutée, sous forme de bille extrudée dans une solution acide de chitosan contenant du $CaCl_2$ (réticulant de l'alginate). Les billes sont ensuite exposées à un vide pour en modifier la porosité. L'utilisation de glutaraldéhyde permet la réticulation de la gélatine ou du chitosan par l'intermédiaire de liaisons covalentes. Cependant le glutaraldéhyde est toxique pour les cellules. L'ajout du chitosan dans un deuxième temps aboutit à la formation d'une coque autour des microsphères et à une répartition non homogène de celui-ci au sein de la structure du matériau final. Enfin, la simple utilisation du bicarbonate de sodium aboutit à la formation de bulles et donc à une porosité non interconnectée dans les billes. L'étape de séchage sous vide utilisée en fin de procédé provoque l'interconnexion des pores *via* la rupture des parois les plus fines de la structure poreuse mais conduit à une population de pores aux dimensions aléatoires et qui ne sont pas obligatoirement adaptées à des applications de culture cellulaire.

Il existe donc un besoin pour des matrices polymères biocompatibles et biodégradables qui soient hautement poreuses tout en présentant de bonnes propriétés mécaniques.

Les inventeurs se sont donc donné pour but de mettre au point un procédé de préparation permettant d'accéder à des matrices polymères tridimensionnelles, biocompatibles et biodégradables qui allient à la fois de bonnes propriétés de résistance mécanique tout en comportant un réseau de pores ouverts et interconnectés répondant aux critères de capacité d'ensemencement, de biocompatibilité et de biomimétisme afin de permettre leur utilisation comme support de cellules et/ou pour la culture de cellules, ainsi qu'en thérapie tissulaire, notamment en thérapie tissulaire cardiaque.

La présente invention a donc pour premier objet un procédé de préparation d'une matrice polymère biocompatible et biodégradable comportant un réseau de pores ouverts et interconnectés, ledit procédé comportant au moins les étapes suivantes :

1) la préparation d'une solution aqueuse comprenant au moins un polysaccharide anionique biocompatible et au moins un polymère cationique biocompatible,

2) l'agitation mécanique de ladite solution obtenue ci-dessus à l'étape précédente en présence d'un agent moussant ou d'un gaz sous pression, pour former une mousse,

3) la congélation de la mousse obtenue ci-dessus à l'étape précédente, pour obtenir une mousse congelée,

4) la gélification de la mousse congelée obtenue ci-dessus à l'étape précédente, par ajout à ladite mousse d'au moins un agent gélifiant en solution dans un solvant, pour obtenir une mousse gélifiée,

5) la déshydratation de la mousse gélifiée obtenue ci-dessus à l'étape précédente, pour obtenir une mousse gélifiée déshydratée, puis

6) le séchage de la mousse gélifiée déshydratée obtenue ci-dessus à l'étape précédente, par traitement au $CO_2$ supercritique, pour obtenir ladite matrice polymère.

Grâce au procédé conforme à l'invention, il est maintenant possible de préparer des matrices polymères biocompatibles et biodégradables comportant un réseau de pores ouverts et interconnectés permettant leur utilisation pour la culture de cellules et ayant en outre une résistance mécanique ainsi qu'une élasticité suffisante pour permettre leur utilisation en thérapie cellulaire, notamment en thérapie cellulaire cardiaque.

Selon l'invention, le terme « biocompatible » utilisé dans la présente description pour qualifier ladite matrice polymère ou une substance, telle que par exemple un polysaccharide anionique ou un polymère cationique, signifie que ledit matériau ou ladite substance n'interfère pas et ne dégrade pas le milieu biologique dans lequel il/elle est utilisé(e).

Au sens de la présente invention, l'expression « pores ouverts et interconnectés » signifie que la matrice polymère possède une porosité ouverte, c'est-à-dire une porosité à laquelle on accède depuis l'extérieur de la matrice, et que lesdits pores communiquent entre eux pour former un réseau tridimensionnel.

Le ou les polysaccharides anioniques biocompatibles utilisables selon l'invention ont de préférence une masse moléculaire moyenne ($Mw_A$) supérieure ou égale à 75 000 Daltons, de préférence allant de 75 000 à 250 000 Daltons environ, et encore plus préférentiellement de 140 000 à 240 000 Daltons environ.

Le ou les polysaccharides anioniques biocompatibles utilisables selon l'invention sont de préférence choisis parmi les alginates et alginates modifiés, les pectines, les dérivés cellulosiques, les gommes polysaccharidiques telles que l'agar-agar, la gomme xanthane et la gomme gellane, les carraghénanes, les dextranes modifiés et l'acide hyaluronique.

Selon une forme de réalisation préférée de l'invention, le ou les polysaccharides anioniques biocompatibles sont choisis parmi les alginates et les alginates modifiés.

Parmi les alginates, on préfère tout particulièrement les alginates comprenant de 32 à 61 % d'unités acide mannuronique (M), de 39 à 68 % d'unités acide guluronique (G) par rapport au nombre total d'unités constituant lesdits alginates, et dans lequel le ratio M/G varie de 0,47 à 1,56.

Parmi de tels alginates, on peut notamment mentionner les alginates de sodium comportant au moins 60 % en nombre d'unités G tels que les produits vendus sous les dénominations commerciales Pronova UP MVG®, Pronova UP LVG®, Alginate SLG20® et Alginate SLG100® par la société Novamatrix, les alginates comportant au moins 50 % en nombre d'unités M tels que les produits vendus sous les dénominations commerciales Pronova UP MVM®, Pronova UP LVM®, Alginate SLM20® et Alginate SLM100® par la société Novamatrix, ou bien encore les alginates de sodium vendus sous les dénominations commerciales Alginate Medium Viscosity et Alginate High Viscosity par la société Sigma-Aldrich.

Selon une forme de réalisation tout particulièrement préférée de l'invention, le polysaccharide anionique biocompatible est un alginate de sodium ayant une viscosité supérieure ou égale à 2000 mPa-s et une masse moléculaire moyenne comprise entre 80 000 et 120 000 Da inclusivement.

Au sens de la présente invention, on entend par « alginate modifié », un alginate dont la structure de base est fonctionnalisée par un ou plusieurs groupements, en particulier par un ou plusieurs peptides tels que par exemple des tripeptides composés de L-arginine, de glycine, et d'acide L-aspartique (peptides RGD), qui sont des peptides impliqués dans l'adhésion cellulaire. A titre d'exemple, de tels alginates modifiés sont par exemple vendus sous les dénominations commerciales Novatach®G RGD, Novatach®M RGD, Novatach®G VAPG et Novatach®M REGV par la société NovaMatrix.

Selon une forme de réalisation préférée de l'invention, la quantité de polysaccharides anionique présents au sein de la solution aqueuse de l'étape 1) varie de 0,5 à 8 % en masse environ, et encore plus préférentiellement de 1 à 3 % en masse environ par rapport à la masse totale de ladite solution aqueuse.

Le ou les polymères cationiques biocompatibles utilisables selon la présente invention ont de préférence une masse moléculaire moyenne ($Mw_C$) supérieure ou égale à 100 000 Daltons, plus préférentiellement allant de 150 000 à 600 000 Daltons environ, en encore plus préférentiellement de 190 000 à 310 000 Daltons environ.

Le ou les polymères cationiques biocompatibles utilisables selon la présente invention sont de préférence choisis parmi les polysaccharides cationiques, en particulier dans le groupe comprenant le chitosan, des formes salines particulières du chitosan et les dérivés du chitosan ; et les polymères ayant des groupes réactifs basiques tels que des groupes amine ou imine parmi lesquels on peut citer les polyéthylèneimines, les poly(L-lysines), les poly(vinylamines), les poly(aminoacides) et les poly(alkylamines).

Le ou les polymères cationiques biocompatibles sont de préférence choisis parmi le chitosan, des formes salines particulières du chitosan et les dérivés de chitosan. On peut en particulier mentionner les chitosans vendus sous les dénominations commerciales Chitosan Low Molecular Weight, Chitosan Medium Molecular Weight et Chitosan High Molecular Weight, Chitosan High Purity Mw 60,000-120,000, Chitosan High Purity Mw 110,000-150,000 et Chitosan High Purity Mw 140,000-220,000 par la société Sigma-Aldrich, ou bien encore Protasan UP CL 113, 114, 213 et 214 par la société NovaMatrix. Parmi ces chitosans, on préfère tout particulièrement les chitosans ayant une viscosité de 200 à 800 mPa-s environ, une masse moléculaire moyenne comprise entre 190 000 et 310 000 Da inclusivement et un taux de désacétylation supérieur à 80 %.

Les formes salines particulières du chitosan (pouvant également être dénommées sels du chitosan) peuvent notamment être choisies parmi les chlorure, lactate, acétate et glutamate de chitosan.

Au sens de la présente invention, on entend par « dérivé du chitosan », un chitosan dont la structure de base est fonctionnalisée par un ou plusieurs groupements fonctionnels, en particulier, par un ou plusieurs groupements choisis parmi les groupements carboxyméthyle, hydroxybutyle ou bien encore un groupement glycérylphosphate-hydroxyéthylcellulose. A titre d'exemples de tels dérivés du chitosan, on peut notamment mentionner les produits vendus sous la dénomination commerciale Chitoscience® ou Chitoceuticals Carboxymethylchitosan® par la société HMC+.

Selon une forme de réalisation préférée de l'invention, la quantité de polymères cationiques présents au sein de la solution aqueuse de l'étape 1) varie de 0,5 à 15 % en masse environ, et encore plus préférentiellement de 1 à 2,25 % en masse environ par rapport à la masse totale de ladite solution aqueuse.

Selon une forme de réalisation préférée de l'invention, le rapport en masse polysaccharides anioniques ($M_{PA}$) / polymères cationiques ($M_{PC}$) présents dans la solution aqueuse de l'étape 1) varie de 20/80 à 80/20, et encore plus préférentiellement de 40/60 à 60/40 environ.

La concentration totale en polymères présents au sein de la solution aqueuse de l'étape 1), c'est-à-dire la somme des quantités en polysaccharides anioniques et en polymères cationiques, varie de préférence de 1 à 10 % en masse environ, et encore plus préférentiellement de 1,5 à 3,75 % en masse environ par rapport à la masse de la solution aqueuse.

Outre le ou les polysaccharides anioniques et le ou les polymères cationiques, la solution aqueuse préparée lors de l'étape 1) comprend de préférence au moins un tensioactif hydrophile dont la présence permet de stabiliser la mousse formée à l'étape 2).

Au sens de la présente invention, on entend par tensioactif hydrophile, un tensioactif présentant une valeur de HLB (« *Hydrophilic-Lipophilic Balance* » : balance hydrophile/hydrophobe) supérieure ou égale à 8, et de préférence supérieure ou égale à 12. Leur présence dans la solution aqueuse préparée à l'étape 1) permet de stabiliser la mousse formée lors de l'étape 2).

Le ou les tensioactifs hydrophiles sont de préférence choisis parmi les tensioactifs non-ioniques.

Parmi de tels tensioactifs non-ioniques, on peut en particulier mentionner les esters d'acide gras et de sorbitan éthoxylés (polysorbates) tels que les produits vendus sous les dénominations commerciales Eumulgin®SML20, SMS20 par la société BASF, Montanox®20 PPI, Montanox® 20 API et Montanox®80 PPI par la société Seppic, Alkest® TW20, TW60, TW80, TW80 K, TW327 par la société Univar, Carnacel® TW20 et TW80 par la société Quimica Delta, et Tween® 20, 40, 60, 65, 80 et 85 par la société Sigma-Aldrich ; les copolymères blocs nonioniques polyoxyéthylène-polyoxypropylène(également appelés poloxamères) tels que les produits vendus sous les dénominations commerciales Pluronic®, en particulier Pluronic® F68, Pluronic® F108 et Pluronic® F127, par la société Sigma-Aldrich, ou Synperonic® ou Kolliphor®également par la société Sigma-Aldrich ; les dérivés de cellulose tels que l'hydroxypropylméthylcellulose ; les glucosides et les alcanolamides.

Le ou les tensioactifs hydrophiles peuvent également être choisis parmi les tensioactifs anioniques, et en particulier parmi le dodécylsulfate de sodium tel que les produits vendus sous les dénominations commerciales Triton ®, en particulier Triton® X-405 par la société Sigma-Aldrich, et le bromure de cétyl triméthylammonium (connu sous l'acronyme CTAB).

Plus rarement, le ou les tensioactifs hydrophiles peuvent être choisis parmi certains polymères cationiques tels que par exemples les polyquaterniums, comme en particulier le Polyquaternium-10.

Enfin, des protéines telles que l'albumine et la gélatine peuvent aussi jouer le rôle de tensioactif hydrophile.

Selon une forme de réalisation préférée de l'invention, le tensioactif hydrophile est un tensioactif nonionique, et encore plus préférentiellement un polysorbate ou un poloxamère.

Le ou les tensioactifs hydrophiles représentent de préférence de 0,01 à 5 % en masse, et encore plus préférentiellement 1 % en masse environ, par rapport à la masse totale de la solution aqueuse de l'étape 1).

Bien que cela ne soit pas obligatoire, la solution aqueuse préparée à l'étape 1) peut en outre renfermer un ou plusieurs agents plastifiants.

Dans ce cas, le ou les agents plastifiants sont de préférence choisis parmi le glycérol, le sorbitol et leur mélange.

Lorsqu'ils sont utilisés, le ratio du ou des agents plastifiants par rapport aux polymères en solution aqueuse préparée à l'étape 1) peut varier de 10:1 à 2:1, préférentiellement de 8:1 à 3:1, et encore plus précisément de 6:1 à 4:1.

L'agitation mécanique effectuée lors de l'étape 2) est de préférence réalisée à une vitesse de rotation supérieure ou égale à 1500 tours par minute (tpm), et encore plus préférentiellement à une vitesse de rotation allant de 1500 à 2100 tpm environ.

La durée de l'agitation mécanique varie généralement de 5 à 120 minutes environ. Typiquement elle est d'environ 30 minutes.

L'agitation mécanique est de préférence réalisée à l'aide d'un appareil à pales classique.

Selon une première forme de réalisation de l'étape 2), la mousse est formée en présence d'un agent moussant qui est alors ajouté à la solution préparée à l'étape 1) juste avant la réalisation de l'étape 2).

Dans ce cas, l'agent moussant (également dénommé « agent porogène » ou « agent générateur de gaz ») est de préférence choisi parmi le bicarbonate de sodium et le carbonate de sodium, et la solution aqueuse de l'étape 1) est alors porté à un pH acide, variant de préférence de 4,0 à 6,5 par ajout d'au moins un agent acidifiant.

Selon cette première forme de réalisation de l'étape 2), l'agent moussant représente alors de préférence de 0,5 à 5 % en masse environ, et encore plus préférentiellement de 1 à 2 % en masse environ, par rapport à la masse totale de la solution aqueuse préparée à l'étape 1).

L'ajout d'un agent acidifiant permet d'obtenir un dégagement gazeux à partir de l'agent moussant présent dans la solution aqueuse.

L'agent acidifiant peut par exemple être choisi parmi l'acide acétique, l'acide adipique, l'acide tartrique, la glucono-$\delta$-lactone et le peroxyde d'hydrogène.

Dans ce cas, l'agent acidifiant (sauf peroxyde d'hydrogène) représente de préférence de 0,05 à 15 % en masse environ par rapport à la masse totale de la solution aqueuse. Si l'agent acidifiant est le peroxyde d'hydrogène, alors sa concentration peut aller jusqu'à 40 % environ en masse par rapport à la masse totale de la solution aqueuse.

Selon une seconde forme de réalisation de l'étape 2), la mousse est formée par introduction d'un gaz sous pression dans la solution aqueuse préparée à l'étape 1).

Dans ce cas, le gaz est par exemple choisi parmi l'air, l'argon et le dioxyde de carbone.

Selon cette seconde forme de réalisation, la pression à laquelle est introduit ledit gaz peut varier de 1 à 100 bars environ.

La durée d'introduction dudit gaz sous pression dans la solution aqueuse peut varier de 20min à 2 h environ.

La congélation de l'étape 3) est de préférence réalisée en portant la mousse obtenue à l'étape 2) à une température inférieure ou égale à -10°C environ, et encore plus préférentiellement à une température allant de -18 à -20°C environ.

Selon une forme de réalisation particulière et préférée, l'étape 3) de congélation est réalisée en maintenant la mousse obtenue à l'étape 2) à une température d'environ -18°C pendant 8 à 24 heures.

Selon une autre forme de réalisation particulière, l'étape 3) de congélation est réalisée en trempant la mousse obtenue à l'étape 2) dans un bain d'azote liquide à une température de -180°C. Dans ce cas, la congélation est rapide, de l'ordre de quelques secondes.

Lors de l'étape de congélation, la mousse obtenue à l'étape 2) peut être introduite dans un moule de façon à conférer une forme particulière à ladite mousse, après congélation.

Selon une forme particulière de réalisation du procédé conforme à l'invention, l'étape 3) de congélation peut être suivie d'une étape 3bis) de lyophilisation de la mousse congelée obtenue à l'issue de l'étape 3).

Dans ce cas, l'étape 3bis) de lyophilisation peut être réalisée sous vide à une température allant de -40 à -50°C environ et à une pression allant de 10 à 100 $\mu$m de mercure environ.

L'agent gélifiant utilisé lors de l'étape 4) de gélification de la mousse congelée est de préférence une solution d'au moins un sel d'un cation divalent ou trivalent dans un solvant. De tels cations sont de préférence choisis parmi les cations inorganiques tels que le cuivre, le calcium, l'aluminium, le magnésium, le strontium, le baryum, le zinc, le chrome, ainsi que parmi les cations organiques tels que les sels d'alkylammonium, la polyéthylèneimine, la poly(vinylamine), les poly(aminoacides) et les poly(alkylamines).

Le solvant de la solution d'agent gélifiant est de préférence l'eau, tamponnée ou non.

Selon une forme de réalisation préférée de l'invention, l'agent gélifiant est une solution aqueuse de chlorure de calcium, de chlorure de strontium, de gluconate de calcium, de carbonate de calcium, ou de carbonate de strontium.

La concentration de la solution d'agent gélifiant peut varier de 0,005 M à 1 M environ, et de préférence de 0,05 à 1 M environ.

A l'issue de l'étape 4), la mousse est de préférence lavée plusieurs fois de façon à éliminer le tensioactif, par exemple à l'aide d'une solution à pH neutre telle qu'une solution tampon, par exemple un tampon à base d'acide 4-(2-hydroxyéthyl)-1-pipérazine éthane sulfonique (tampon HEPES).

L'étape 5) de déshydratation de la mousse gélifiée obtenue à l'étape 4) est de préférence réalisée de façon progressive, par immersion de ladite mousse gélifiée dans des bains successifs d'éthanol absolu de concentrations croissantes. A titre d'exemple, la déshydratation de la mousse gélifiée peut être faite par immersions dans 3 bains d'éthanol absolu à 20 %, puis 3 bains d'éthanol absolu à 40 %, puis 3 bains d'éthanol absolu à 80 %, puis pour finir, 3 bains dans l'éthanol absolu à 100 %, chacune des immersions ayant une durée de 10 à 15 minutes environ.

L'étape 6) de séchage au $CO_2$ supercritique de la mousse déshydratée obtenue à l'issue de l'étape 5) est de préférence réalisée à une température allant de 35 à 50°C environ, et à une pression allant de 45 à 95 bars environ.

Selon une forme de réalisation particulière et préférée, l'étape de séchage au $CO_2$ supercritique de la mousse déshydratée est réalisée à une température d'environ 44°C, sous environ 85 bars de pression, pendant environ 25 minutes.

Lorsque l'étape 6) de déshydratation est terminée, la matrice polymère est prête à être utilisée ou stockée pour une utilisation ultérieure. Avant utilisation, ladite matrice est de préférence stérilisée selon les méthodes bien connues de l'homme du métier et telles que par exemple l'exposition aux rayons UV, la $\gamma$-irradiation, l'utilisation de faisceaux d'électrons pulsés ou non, d'oxyde d'éthylène, l'autoclavage ou le contact avec un alcool ou avec un gaz de formule NOx, la stérilisation par gaz plasma (Sterrad®) pour autant qu'aucune de ces méthodes n'affecte les propriétés de la matrice finale ou les caractéristiques de ces composants.

La matrice polymère biocompatible et biodégradable obtenue par la mise en œuvre du procédé tel que défini selon le premier objet de l'invention, est nouvelle en soi et constitue à ce titre le deuxième objet de l'invention.

Une matrice polymère biocompatible et biodégradable est obtenue par la mise en œuvre du procédé conforme à la présente invention, ladite matrice étant caractérisée en qu'elle se présente sous la forme d'un matériau alvéolaire constitué d'une matrice polymère poreuse résultant de la gélification d'une mousse d'au moins un polysaccharide anionique biocompatible et d'au moins un polymère cationique biocompatible, et en ce que ladite matrice :

- comprend des pores ouverts et interconnectés ayant une dimension moyenne $d_A$ allant de 0,2 $\mu$m à 400 $\mu$m ;
- présente un volume poreux allant de 60 à 98 % du volume total de la matrice ;
- présente un module d'élasticité à 50 % de déformation ($E_{50\%}$) variant de 1 à 100 kPa.

Le module d'élasticité à 50 % de déformation $E_{50\%}$ est déterminé à l'aide d'un texturomètre vendu sous la dénomination commerciale TA-XT2 Texture Analyser par la société Stable Micro Systems, avec un piston cylindrique en Plexiglas® (poly(méthacrylate de méthyle) d'un diamètre de 20 mm et une vitesse de compression de 2 mm/s pour mesure la force nécessaire pour comprimer un échantillon de matrice polymère à 50 % de sa hauteur initiale. Le module d'élasticité à 50% de déformation est ensuite calculé en appliquant la formule suivante :

$$E_{50\%} = \frac{(F_{50\%}/S)}{0,5} \times 1000$$

dans laquelle $E_{50\%}$ et $F_{50\%}$ sont respectivement le module d'élasticité (en KPa) et la force (en N) nécessaire pour obtenir 50 % de déformation et S est la surface de l'échantillon de matrice polymère (en mm²) au contact du piston. Une telle méthode a par exemple été décrite par Shapiro L et al. (Biomaterials 1997,18, 583-590).

Selon une forme de réalisation préférée de ce matériau, le module d'élasticité en compression varie de 30 à 45 kPa.

Par ailleurs, ladite matrice polymère à l'état réhydraté présente de préférence un module de Young en traction variant de 0,3 à 20 kPa, préférentiellement de 0,5 à 15 kPa et encore plus préférentiellement de 1 à 10 kPa. Les mesures de résistance à la traction des matrices polymères conformes à l'invention ont été réalisées après réhydratation d'échantillons en forme d'haltères dans un milieu « *Minimum Essential Medium* » (MEM) alpha complet (MEM α complet) jusqu'à gonflement complet. Les mesures ont été réalisées à l'aide d'un texturomètre TAXT2 équipé de mors, selon le protocole décrit dans l'article de Andersen et al., Biomacromolecules, 2012 (Norme ASTMD638-10 (Type I)).

Enfin, ladite matrice polymère présente de préférence des modules de conservation (G') et de perte (G") allant de 100 à 25 000 Pa.

G* est une grandeur rhéologique appelée module complexe. Lorsqu'on soumet un matériau à un test dynamique de cisaillement, on constate qu'il y a un déphasage entre la contrainte appliquée et la déformation du matériau. Cela traduit des phénomènes visco-élastiques de conservation d'énergie (exprimés *via* le module G', appelé module de conservation) et de dissipation d'énergie (exprimés *via* le module G" appelé module de perte). Les modules G' et G" sont mesurés lors d'un test rhéologique en mode dynamique (oscillatoire) selon la méthode décrite par Kong H.J. et al. (Polymer, 2002, 43, 6239-6246).

Egalement selon une forme de réalisation préférée de ce matériau, les modules (G') et (G") varient de 12 000 à 25 000 Pa pour G' et de 1000 à 3000 kPa pour G".

Comme indiqué précédemment, la nature même du matériau (matrice polymère biocompatible à haute porosité), rend son utilisation pour la culture de cellules particulièrement avantageuse.

Par conséquent, la matrice polymère biocompatible et biodégradable telle qu'obtenue par le procédé tel que défini selon l'objet de l'invention peut être utilisée comme support de cellules animales ou humaines et/ou pour la culture de cellules animales ou humaines *in vitro,* en particulier de cellules indifférenciées de mammifères telles que par exemple des cellules souches mésenchymateuses.

En effet, les études effectuées par les inventeurs ont montré qu'une telle matrice constitue un environnement poreux favorable à la viabilité et au maintien des fonctionnalités des cellules souches. De plus, il est possible de faire varier la porosité et les propriétés mécaniques des matrices de l'invention dans les gammes indiquées ci-dessus en fonction du choix de la nature des polymères utilisés pour sa préparation et du type de cellules que l'on souhaite supporter ou cultiver.

Ainsi, une matrice polymère poreuse renfermant des cellules animales ou humaines, en particulier des cellules indifférenciées de mammifère, peut être obtenue, ledit support étant caractérisé en ce que la matrice polymère poreuse est une matrice telle qu'obtenue selon le procédé tel que défini selon l'objet de l'invention, et en ce que lesdites cellules sont majoritairement présentes dans les pores de ladite matrice.

L'excellente biocompatibilité et les propriétés mécaniques d'un tel support de cellules rendent son utilisation en thérapie tissulaire, et en particulier en thérapie tissulaire cardiaque particulièrement avantageuse.

Ainsi, le support de cellules peut être utilisé en médecine régénérative, notamment en thérapie cellulaire, en particulier en thérapie cellulaire cardiaque.

Un tel support permet, après implantation, notamment au niveau d'une lésion du muscle cardiaque, de localiser les cellules sur le site d'implantation, tout en les protégeant lors de l'implantation et en les maintenant à l'état indifférencié de façon à prolonger leurs effets paracrines.

La présente invention est illustrée par les exemples de réalisation suivants, auxquels elle n'est cependant pas limitée.

## EXEMPLES

Les matières premières utilisées dans les exemples qui suivent sont listées ci-après :

- Alginate de sodium d'algue brune, de masse moléculaire moyenne comprise entre 80 000 et 120 000 Da, et de viscosité ≥ 2000 mPa-s (à 2% massique dans l'eau, et à 25°C), vendu sous la dénomination commerciale Alginic acid sodium salt from brown algae - Medium viscosity par la société Sigma-Aldrich ;
- Chitosan désacétylé à 80 %, de masse moléculaire moyenne comprise entre 190 000 et 300 000 Da, de viscosité comprise entre 200 et 800 mPa-s (à 1 % massique dans l'acide acétique à 1% et à 25°C), vendu sous la dénomination commerciale Chitosan medium molecular weight par la société Aldrich ;
- NaCl et carbonate de calcium (société BDH Prolabo)
- acide acétique pur (société Fisher Chemical)
- éthanol absolu, tampon HEPES (société Sigma-Aldrich)

### Caractérisations physico-chimiques

Microscopie électronique à balayage (MEB) :

Les matrices polymères préparées dans les exemples qui suivent ont été métallisées à l'argent par pulvérisation cathodique à l'argon à l'aide d'un appareil vendu sous la dénomination commerciale Sputter Coater S 150B® par la société Edwards, puis observées à l'aide d'un microscope électronique à balayage JSM-6400 de la société Jeol sous une tension de 10 kV. Pour chaque échantillon, 10 mesures du diamètre des pores ont été effectuées en surface et en coupe transversale puis la moyenne a été calculée.

Microscopie électronique à balayage environnementale :

Des échantillons cubiques de dimensions approximatives 5x5x2,5 mm, ont été découpés à partir des matrices polymères préparées dans les exemples qui suivent et introduits à l'état sec dans la chambre d'un microscope ESEM Quanta® 250 FEG de la société FEI. Les échantillons y ont été hydratés progressivement en contrôlant le taux d'humidité dans la chambre qui augmente progressivement de 85 à 99 % en régulant la pression de vapeur d'eau. La tension d'accélération a été réglée à 15 kV et la température à 2°C.

Résistance mécanique (Essai de compression uniaxiale) :

Des essais de compression uniaxiale ont été effectués sur des échantillons des matrices polymères préparées dans les exemples qui suivent, après hydratation pendant 24 heures dans un milieu de culture cellulaire. Chaque échantillon a été soumis à 3 essais de compression uniaxiale et les mesures ont été faites en triple. Les mesures ont été réalisées à l'aide d'un texturomètre vendu sous la dénomination commerciale TA-XT2 Texture Analyser par la société Stable Micro Systems, avec un piston cylindrique en aluminium d'un diamètre de 20 mm et une vitesse de compression de 2 mm/s pour mesure la force nécessaire pour comprimer les échantillons à 50 % de leur hauteur initiale. Le module d'élasticité à 50% de déformation a ensuite été calculé en appliquant la formule suivante :

$$E_{50\%} = \frac{(F_{50\%}/S)}{0,5} \times 1000$$

dans laquelle $E_{50\%}$ et $F_{50\%}$ sont respectivement le module d'élasticité (en KPa) et la force (en N) nécessaire pour obtenir 50 % de déformation et S est la surface de l'échantillon (en $mm^2$) au contact du piston.

Résistance mécanique (Essais en traction uniaxiale) :

Dans les exemples qui suivent, des essais de traction ont été effectués sur des échantillons des matrices polymères en forme d'haltères (selon la Norme ASTM D638-10 (Type I)), après réhydratation pendant 24 heures dans un milieu de culture cellulaire. Le module d'Young des échantillons hydratés a été déterminé en traction uniaxiale à l'aide d'un texturomètre vendu sous la dénomination commerciale TA-XT2 Texture Analyser par la société Stable Micro Systems, avec une vitesse constate de 0,5 mm/s pour mesurer la force nécessaire jusqu'à la rupture. La courbe de la contrainte (égale à la force en Newtons rapportée à la surface en $mm^2$) en fonction de la déformation (en %) est tracée et le module d'Young est ensuite calculé comme étant la pente à l'origine dans la partie linéaire de cette courbe. Les mesures sont faites sur 3 à 5 échantillons pour chaque ratio alginate/chitosan. Une telle méthode est décrite par exemple dans l'article de Andersen *et al.,* 2012, Biomacromolecules.

Cultures de cellules souches :

Des cultures de cellules souches mésenchymateuses de moelle osseuse de rat (CSMr) ont été réalisées de la façon suivante :
Un milieu de culture de cellules « *Minimum Essential Medium* » (MEM) alpha complet (MEM α complet) a été préparé en mélangeant 450 mL de milieu MEM α, GlutaMAX® vendu sous la référence 32561 par Gibco, 5 mL d'un mélange Pénicilline/Streptomycine (10000 U /mL) vendu sous la référence 15140 par Gibco et 50 mL de Sérum de veau fœtal vendu sous la référence A15-043 par PAA.

Les CSMr ont été décongelées dans 15 mL de milieu de culture MEM $\alpha$ complet préalablement chauffé à 37°C. Après centrifugation pendant 5 min à 1200 tpm, le surnageant a été aspiré puis le culot de cellules a été repris dans 25 de milieu de culture MEM $\alpha$ complet. Les cellules ont ensuite été ensemencées dans une flasque de culture à la densité de 10 000 cellules/cm$^2$. Le milieu de culture MEM $\alpha$ complet a été changé tous les 2 à 3 jours. Les cellules ont été passées à confluence et réensemencées à la densité de 10 000 cellules/cm$^2$.

Evaluation de la biocompatibilité *in vitro :*

Les CSMr ont été lavées deux fois avec du tampon PBS 1x, puis décollées à la trypsine et comptées. Elles ont ensuite été centrifugées pendant 5 min à 1200 tpm, puis le culot de cellules a été repris dans du milieu de culture MEM $\alpha$ complet. 15 $\mu$L de suspension cellulaire contenant 100 000 CSMr ont été déposés sur les échantillons des matrices polymères à l'état sec dans une plaque de 48 puits, centrifugés pendant 1 min à 400 g et à 25°C afin d'obtenir un ensemencement homogène en profondeur et enfin hydratés dans du milieu de culture MEM $\alpha$ complet. Les plaques ont été maintenues en conditions de culture à 37°C dans une atmosphère à 5 % de $CO_2$.

Evaluation de la viabilité cellulaire au sein des matrices polymères :

- Sérum physiologique : Solution de NaCl à 0,9 % en masse dans l'eau désionisée,

- Marqueurs fluorescents : kit d'essai de la viabilité et de la cytotoxicité vendu sous la dénomination utilisant la calcéine AM et l'Ethidium-III, vendu sous la dénomination « *ViabilityIytotoxicity Assay Kit for Live & Dead cells* »*,* référence FP-BF4710 par la société Interchim Fluo Probes$^®$, France.

Une solution de marquage des cellules a été préparée juste avant utilisation en diluant les marqueurs au 1/10 dans un mélange sérum physiologique/milieu de culture MEM $\alpha$ 1/1 (v/v) de façon à obtenir une solution de marqueurs à 2 $\mu$M d'Ethidium-III et 1 $\mu$M de calcéine AM. La solution de marquage a été gardée à l'obscurité jusqu'à utilisation.

Les cellules CSMr ont été lavées 1 fois avec un mélange sérum physiologique/milieu de culture MEM $\alpha$ 1/1 (v/v) puis incubées avec la solution de marquage pendant 30 min à 37°C à l'obscurité. Après incubation, les cellules ont été lavées au sérum physiologique et conservées dans le sérum physiologique à 37°C jusqu'à observation.

L'observation du marquage a été réalisée à l'aide d'un microscope confocal Zeiss 780 : longueur d'onde d'excitation de la calcéine AM : 495 nm ; longueur d'onde d'émission de la calcéine AM : 515 nm ; longueur d'onde d'excitation de l'Ethidium-III : 495 nm, longueur d'onde d'émission de Ethidium-III : 635 nm. Des images successives en profondeur des matrices ont été acquises, puis une reconstruction en 3 dimensions a été obtenue en utilisant le logiciel associé au microscope.

Evaluation de la biocompatibilité *in vivo* :

La biocompatibilité *in vivo* des matrices polymères a été évaluée sur 3 rats femelles de souche Lewis et de poids moyen 200g. Pour l'anesthésie, l'animal a été placé dans une boîte d'induction au gaz et a reçu un mélange gazeux $O_2$ + isoflurane à 4%. Après perte totale des réflexes périphériques, l'animal a été placé sur le dos. L'anesthésie gazeuse a été entretenue à 2 ou 3% d'isoflurane. Une injection de buprénophrine (100$\mu$g/kg) en souscutané a été pratiquée. L'abdomen a été largement rasé, suivi d'une désinfection à l'alcool 70°. Après avoir vérifié la profondeur de l'anesthésie et la perte totale des réflexes périphériques, une incision cutanée a été effectuée afin d'exposer les muscles pectoraux. Un flexible «écarteur» a été mis en place. La matrice polymère a été glissée entre 2 plans musculaires. La poche musculaire a ensuite été fermée par un point de suture à l'aide d'un monofil 7/0 Prolene. L'écarteur a été retiré et une toilette péritonéale a été réalisée. Le plan cutané a été refermé à l'aide du fil à peau 5/0 Ethilon. Toute l'intervention a été réalisée par un chirurgien sous microscope opératoire (Zeiss OPM1 FC).

Evaluation des effets angiogéniques *in vivo* :

Les effets angiogéniques *in vivo* des matrices polymères ont été évalués sur des rats après implantation intramusculaire des matrices. L'implantation des matrices a été réalisée selon le même protocole que celui utilisé ci-dessus pour l'évaluation de la biocompatibilité *in vivo*. La formation de vaisseaux capillaires et de vaisseaux plus matures (artérioles) a été étudiée après 28 jours d'implantation, en immunofluorescence en utilisant des anticorps dirigés contre le facteur de Von Willebrand (VWF) permettant de détecter les cellules endothéliales et contre l'actine alpha des muscles lisses ($\alpha$-SMA) permettant de détecter les muscles des artérioles selon les protocoles ci-après :

**Histologie -Immunomarquages** :

Vingt-huit jours après leur implantation, les matrices ont été prélevées, rincées au sérum physiologique et fixées immédiatement au paraformaldéhyde 4% (dans du PBS 1X, pH 7,4) pendant 48h, puis transférées dans de l'éthanol 70%. Après inclusion en paraffine, des coupes histologiques d'épaisseur 4 à 6 $\mu$m ont été réalisées au microtome. Un marquage histologique hématoxyline-éosine et des marquages immunofluorescents anti- $\alpha$-SMA (smooth muscle actin) et anti-VWF (Von Willebrand Factor) ont été effectués. Pour les marquages en immunofuorescence, les coupes sur lames ont d'abord été déparaffinées dans le xylène (3 bains de 5min), puis réhydratées dans des bains successifs d'éthanol (5min chacun) et enfin dans de l'eau (5min). Un démasquage des sites antigéniques a été effectué dans un tampon Tris (10mM) - EDTA (1mM) contenant 0,05% de Tween20 à 121° pendant 3min. Les échantillons ont ensuite été perméabilisés au Triton (0,5%) et les fonctions aldéhyde du fixateur n'ayant pas réagi ont été neutralisées dans une solution de glycine 0,1 M (2 bains de 10min chacun). Les sites antigéniques non-spécifiques ont été saturés avec une solution tampon de PBS contenant 2% de sérum de chèvre, 1% d'albumine de sérum bovin et 0,2% de Triton (30min). Les lames ont ensuite été marquées avec un anticorps anti- $\alpha$-SMA (monoclonal mouse anti-alpha-SMA, A2547, Sigma, dilution 1/1000$^e$) et un anticorps anti-VWF (polyclonal rabbit anti-human VW Factor, A0082, Dako, dilution 1/200$^e$) dilués dans la solution tampon de saturation. Après trois lavages (PBS-Tween20 0,2%, 10min chacun), des anticorps secondaires ont été ajoutés : Alexa Fluor® 568 goat anti-mouse (A11019, Life Technologies) et AlexaFluor® 488 goat anti-rabbit (A11008, Life Technologies) pendant 30min à l'obscurité. Après 3 lavages, les noyaux ont été marqués au DAPI (D9542, Sigma, dilution 0,05$\mu$g/ml dans du PBS, 10min). Enfin, les lames ont été lavées et montées avec une lamelle en utilisant une solution de montage pour fluorescence (F4680, Sigma). Toutes les étapes ont été effectuées à température ambiante.

**Observation des immunomarquages par microscopie confocale**

L'observation des immunomarquages a été faite au microscope confocal Zeiss LSM 780 (Carl Zeiss Microscopy) au grossissement x63. Pour chaque animal, le nombre de vaisseaux positifs pour le $\alpha$-SMA dont la lumière est fermée et dont le diamètre est supérieur ou égal à 5$\mu$m a été compté dans la zone de l'implant sur au moins 5 photos non-chevauchantes. Connaissant la surface totale du champ optique (en mm$^2$), la densité d'artérioles a été calculée comme étant égale au nombre de vaisseaux/mm$^2$.

**Statistiques sur les immunomarquages**

Pour la comparaison du nombre de vaisseaux par unité de surface entre les groupes implantés (matrices L+ ou référence, acellulaires ou contenant des CSM), le test *t* de Student bilatéral a été utilisé. L'analyse statistique a été effectuée avec le logiciel vendu sous la dénomination commerciale GraphPadPrism version 4 (PrismGraphPad, San Diego, CA). La distribution Gaussienne des données a été testée avec un test de normalité et les résultats ont été exprimés par leur moyenne $\pm$ erreur-type de la moyenne. Un test est considéré comme significatif si la p-value est inférieure à 0,05.

**EXEMPLE 1**

**Préparation de matrices polymères poreuses conformes à la présente invention et de matrices polymères poreuses comparatives ne faisant pas partie de l'invention - Caractérisations**

Dans cet exemple, on a préparé des matrices polymères poreuses conformes à l'invention à base d'alginate à titre de polysaccharide anionique et de chitosan à titre de polymère biocompatible cationique, en utilisant différents ratios massiques alginate/chitosan selon le Tableau 1 ci-après :

**TABLEAU 1**

| | Matrice M100/0 (*) | Matrice M60/40 | Matrice 50/50 | Matrice 40/60 | Matrice 0/100 (*) |
|---|---|---|---|---|---|
| Ratio massique alginate/chitosan | 100/0 | 60/40 | 5050 | 40/60 | 0/100 |
| (*) : Matrices comparatives ne faisant pas partie de l'invention | | | | | |

1) Préparation des matrices polymères

On a préparé les compositions suivantes :

- Solvant de l'alginate (pour 200 g) : 1,8 g de NaCl, compléter à 200 g avec de l'eau désionisée ;

- Solvant du chitosan (pour 100 g) : 0,9 g de NaCl + 1,5 g d'acide acétique pur (soit 0,25 M) et compléter à 100 g avec de l'eau désionisée ;

- Tampon I (pour 1000 g) : 9,0026 g de NaCl + 3,2540 g de tampon HEPES, compléter à 1000 g avec de l'eau Milli-Q® et ajuster le pH à 7,4 avec de l'acide chlorhydrique (HCl) 1M ou 2M.

- Tampon II de gélification (pour 500 mL) : 5 g de chlorure de calcium (soit 0,1 M) + 50 g d'acide acétique pur et compléter à 500 g avec de l'eau Milli-Q® (Merck) puis homogénéiser.

- Agent porogène testé : Sodium bicarbonate : NaHCO$_3$ (société Sigma-Aldrich) - introduit à l'étape 1.

- Tensioactif testé : Polysorbate 20 vendu sous le nom commercial Montanox® 20 DF (société Seppic), introduit à l'étape 1.

Les solutions A, B, C, et D dont les spécifications sont données dans le Tableau 2 suivant ont ensuite été préparées :

**TABLEAU 2**

| Solutions | A | B | C | D |
|---|---|---|---|---|
| Solvant de l'alginate (g) | - | - | - | 200 |
| Poudre d'alginate (g) | - | - | - | 6 |
| Solvant du chitosan (g) | 100 | 100 | 100 | - |
| Poudre de chitosan (g) | 2 | 3 | 4,5 | - |

Les solutions A, B, C et D ont été agitées entre 1600 et 1800 tpm pendant 60 min.

On a ensuite préparé les mousses ayant la composition indiquée dans le Tableau 3 ci-après (pourcentages en masse) selon le protocole décrit précédemment (étapes 1 à 6 du procédé conforme à l'invention) :

**TABLEAU 3**

| Rapport massique alginate/chitosan | Mousse 100/0 (*) | Mousse 60/40 | Mousse 50/50 | Mousse 40/60 | Mousse 0/100 (*) |
|---|---|---|---|---|---|
| Solution A (g) | - | 50 | - | - | - |
| Solution B (g) | - | - | 50 | - | 50 |
| Solution C (g) | - | - | - | 50 | - |
| Solution D (g) | 50 | 50 | 50 | 50 | - |
| Solvant alginate | 50 | - | - | - | - |
| Solvant chitosan | - | - | - | - | 50 |
| % Alginate final | 1,5 | 1,5 | 1,5 | 1,5 | 0 |
| % Chitosan final | 0 | 1 | 1,5 | 2,25 | 1,5 |
| % agent moussant final | 0,9 | 0,9 | 0,9 | 0,9 | 0,9 |
| % Montanox® 20 final | 1 | 1 | 1 | 1 | 1 |
| (*) : Matrices comparatives ne faisant pas partie de l'invention | | | | | |

Les différents ingrédients composant les mousses ont été mélangés et les compositions résultantes ainsi obtenues ont été agitées à 1800 tpm pendant 30 minutes.

Chacune des mousses ainsi obtenues a ensuite été coulée en plaque de 48 puits à raison de 500 μL par puit puis immédiatement congelée à -20°C.

Après congélation, une partie des mousses (appelées « L$^+$ ») a été lyophilisée à une température de -50°C et une pression comprise entre 10 et 100 μm de mercure sous vide (selon l'étape 3bis du procédé conforme à l'invention). Une autre partie des mousses congelées (appelées «L$^-$») n'a pas subi cette étape de lyophilisation.

Les mousses congelées et lyophilisées L$^+$ et les mousses congelées L$^-$ ont ensuite été gélifiées par ajout de 500 μL de tampon II de gélification dans chacun des puits, étant entendu que pour réaliser la gélification de la Mousse 0/100, 500 μl d'une solution de NaOH 1M a été utilisée à la place du tampon II de gélification puisqu'il est connu que la soude utilisée à cette concentration fait gélifier le chitosan.

Au bout d'une heure, les plaques ont été rincées plusieurs fois à l'aide du tampon I de façon à éliminer complètement le tensioactif (3 lavages).

Les mousses gélifiées ont ensuite été déshydratées par immersion des plaques dans des bains successifs de concentration croissante en éthanol absolu : 20 %, 40 % et 80 % à raison de 3 immersions successives de 10 min dans chacun des bains, la dernière déshydratation ayant été réalisée dans un bain d'éthanol absolu à 100 % à raison de 3 immersions successives de 15 minutes.

Les mousses gélifiées et déshydratées ont ensuite été séchées au $CO_2$ supercritique. Pour ce faire, les mousses ont été retirées des plaques 48 puits, placées dans des portes échantillons qui ont été introduits dans la chambre d'un appareil de séchage au $CO_2$ supercritique E3000 Series Critical Point Dryer de la société Quorum Technologies. Le séchage au $CO_2$ supercritique a été réalisé à une température de 44°C sous une pression de 85 bars pendant 25 minutes. La dépressurisation de la chambre a été effectuée à un débit de 2 bars/min jusqu'à atteindre la pression atmosphérique. La chambre a ensuite été ouverte et les matrices attendues ont été récupérées.

2) Résultats des caractérisations

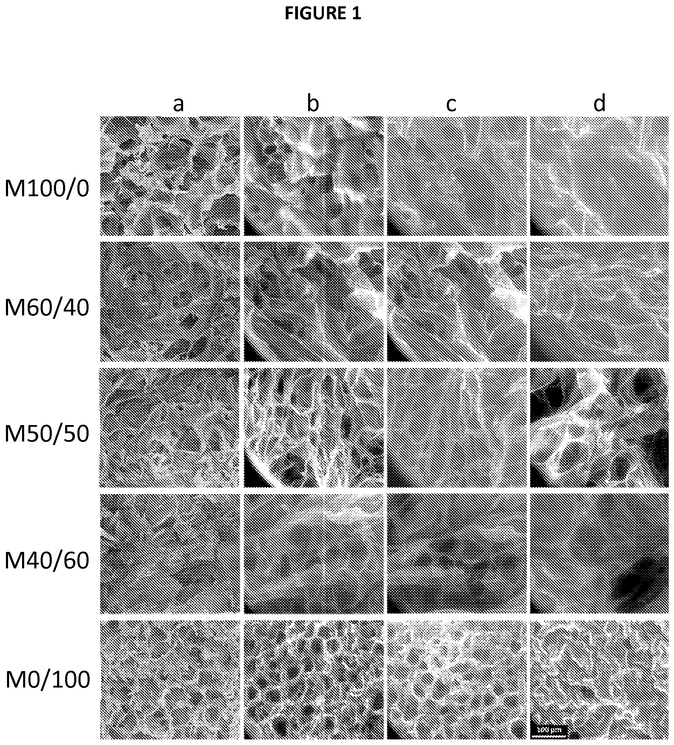

L'aspect macroscopique des matrices ainsi obtenues est montré par la figure 1 annexée qui représente l'aspect microscopique de la structure interne des matrices L$^+$ sèches ou hydratées. Les images des matrices sèches sont obtenues en MEB. Les images des matrices hydratées sont obtenues en MEB environnementale en augmentant progressivement le taux d'humidité de 85 à 99%. La barre d'échelle correspond à 100μm.

Sur cette figure, la figure 1a correspond aux photos MEB des matrices sèches. Les photos des matrices hydratées ont été obtenues en MEB environnementale en augmentant progressivement le taux d'humidité de 85 % (figure 1b) à 99 % (figure 1d), la figure 1c correspondant à un état d'hydratation intermédiaire. Sur la figure 1, la barre d'échelle correspond à 100 μm.

Ces photos montrent dans toutes les matrices présentées la présence de pores interconnectés qui se déploient sous l'effet de la réhydratation ; l'observation microscopique permet de visualiser la porosité ouverte vers l'extérieur des matrices obtenues.

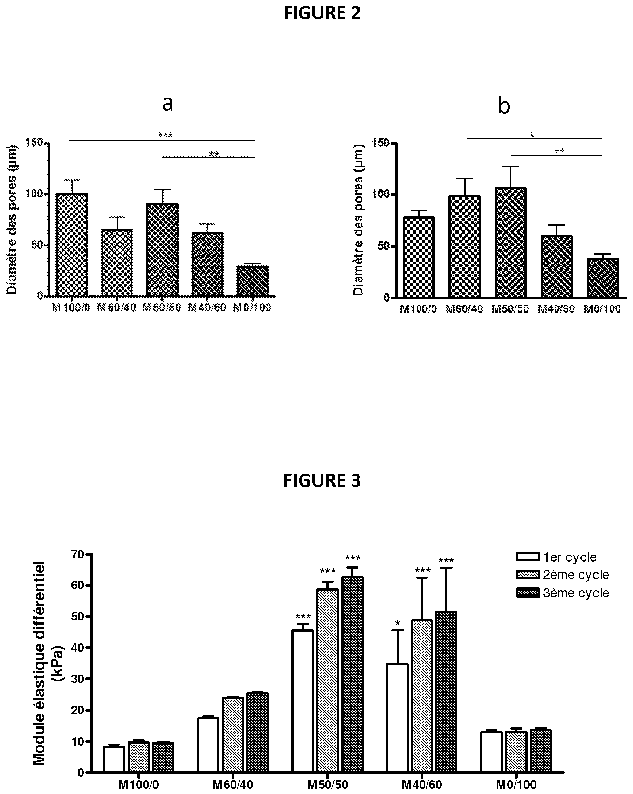

L'évaluation quantitative de la porosité des matrices ainsi obtenues est donnée par la figure 2 annexée : quantification de la taille des pores des matrices sèches L$^+$ (correspondant aux photos sur la figure 1a). Les valeurs affichées sont sous la forme moyenne±écart-type de la moyenne. La valeur de p<0,05 est considérée comme significative. * p<0,05 ; ** p<0,01 ; *** p<0,001.

La porosité de surface (diamètre des pores mesuré au MEB en surface) est reportée sur la figure 2a (diamètre des pores en μm en fonction des matrices préparées). La porosité en coupe (diamètre transversal des pores mesuré au MEB) est reportée sur la figure 2b (diamètre transversal des pores en μm en fonction de matrices préparées).

Les résultats montrent une similitude de porosité entre matrices alginate et matrices alginate/chitosan, qui se montrent globalement supérieure à celle des matrices en chitosan seul, bien qu'elles soient toutes dans le domaine de porosité reconnu comme étant favorable à la survie et la prolifération cellulaire. Les matrices d'alginate et alginate/-chitosan apparaissent donc plus adaptées pour un ensemencement en 3D.

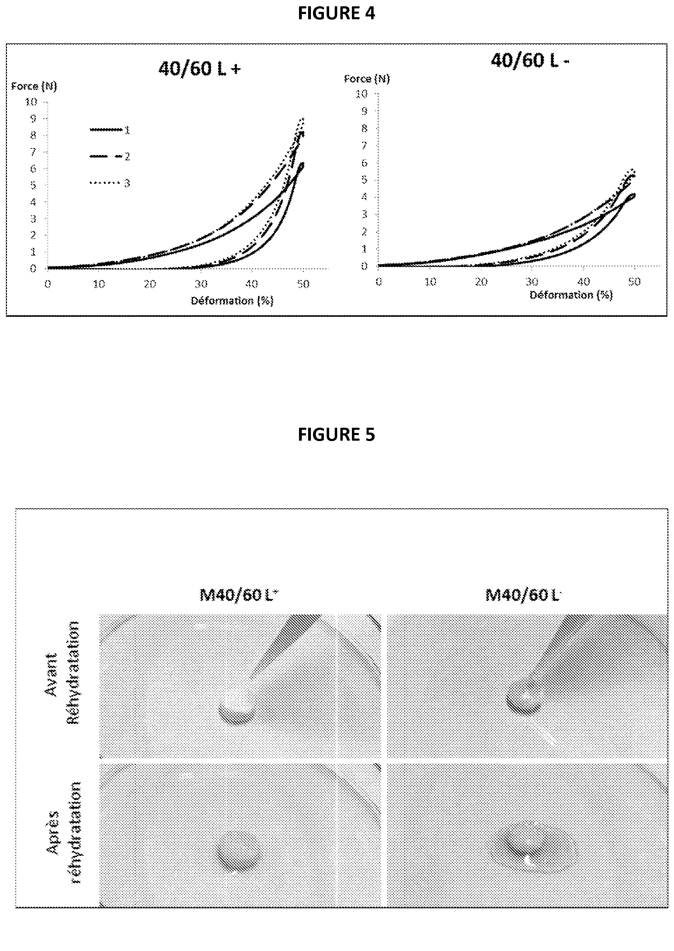

Les résultats de résistance des matrices à la compression sont donnés à la figure 3 annexée, représentant les propriétés mécaniques en compression des matrices L$^+$ hydratées dans du milieu de culture cellulaire soumises à trois cycles de compressions successifs, ainsi qu'à la figure 4 annexée qui compare les résultats obtenus avec la matrice M40/60 L$^+$ ayant subi l'étape intermédiaire 3bis de lyophilisation à ceux obtenus avec la matrice M40/60 L$^-$ n'ayant pas subi ladite étape. En ce qui concerne les résultats présentés à la figure 3, les comparaisons statistiques sont faites versus M100/0.* p<0,05 ; *** p<0,001.

Sur la figure 3, le module élastique différentiel(en kPa) est fonction de la nature des matrices préparées, les barres blanches correspondent au premier cycle de compression, les barres grises au deuxième cycle de compression et les barres noires au troisième cycle de compression.

Sur la figure 4, la force (en Newton, N) est fonction de la déformation (en %), les courbes en trait continu correspondent au premier cycle de compression, les courbe en trait discontinu au deuxième cycle de compression et les courbes en pointillés au troisième cycle de compression.

Les résultats de la figure 3 montrent la synergie liée à l'interaction entre l'alginate et le chitosan, qui aboutit à des matrices aux propriétés mécaniques optimisées comparativement aux matrices à base d'alginate seul (M100/0) ou de chitosan seul (M0/100). La méthode de séchage au $CO_2$ supercritique selon l'étape 6 du procédé conforme à l'invention, a permis l'obtention d'aérogels préservant la porosité de la mousse générée à l'étape 2) puis lors de la gélification à l'étape 4), et les propriétés mécaniques liées à la formation de complexes de polyélectrolytes de charges opposées. La matrice conforme à l'invention dans laquelle le rapport alginate/chitosan est de 50/50 (matrice M50/50) présente les meilleures propriétés de résistance mécanique. A l'inverse les matrices non conformes à l'invention (matrices M0/100 et M100/0) présentent des propriétés mécaniques plus faibles qui peuvent être un frein à leur utilisation pour l'implantation.

Les résultats présentés sur la figure 4 montrent que l'étape 3bis de lyophilisation, bien qu'optionnelle, permet d'améliorer les propriétés mécaniques des matrices conformes à l'invention, la résistance à la compression étant supérieure lorsque la matrice a subi ladite étape.

Les résultats des essais de viabilité des cellules CSMr dans les matrices M0/100, M40/60 et M100/0 après 7 jours de culture sont reportés dans le Tableau 4 ci-après :

**TABLEAU 4**

| Ratio alginate/chitosan | Matrices L⁻ | Matrices L⁺ |
|---|---|---|
| M100/0[(*)] | + | + |
| M40/60 | ++ | ++ |
| M0/100[(*)] | ++ | + |
| (*) : Matrices comparatives ne faisant pas partie de l'invention | | |

Dans le tableau 4, les signes « + » se rapportent à la présence de cellules vivantes (cellules apparaissant en vert à l'observation au microscope confoncal car marquées à la calcéine AM). Le nombre de « + » se rapporte au nombre de cellules vivantes détectables. Toutes les matrices contiennent des cellules vivantes après 7 jours de culture, ce qui démontre la biocompatibilité des matrices conformes à l'invention.

Enfin, il ressort des essais des évaluations de la biocompatibilité après implantation chez le rat (évaluation 1 semaine après implantation intra-musculaire au niveau pectoral) que :

- les matrices obtenues par le procédé conforme à l'invention se prêtent bien à une manipulation chirurgicale et à une implantation sans que cela ne les abîme ;

- l'implantation des matrices n'engendre pas pour les animaux de gêne à se déplacer, à se nourrir, etc. ;

- l'implantation ne provoque pas de réaction inflammatoire massive ni aucune autre réaction physiologique pouvant mettre en danger la santé des animaux implantés ;

- une semaine après leur implantation, les matrices sont maintenues en place (à l'endroit de l'implantation) et conservent leur intégrité (pas de débris de matrice).

## EXEMPLE 2

### Etude du comportement à la réhydratation de deux matrices conformes à la présente invention.

Dans cet exemple on a comparé le comportement à la réhydratation de la matrice M40/60 conforme à l'invention et ayant subi une étape intermédiaire 3bis de lyophilisation, telle que préparée selon le procédé décrit ci-dessus à l'exemple 1 (matrice M40/60 L⁺) à celui de la matrice M40/60 conforme à l'invention mais n'ayant pas subi cette étape intermédiaire 3bis de lyophilisation, telle que préparée également selon le procédé décrit ci-dessus à l'exemple 1 (Matrice M40/60 L⁻).

Pour ce faire, des échantillons de diamètre identique de chacune de ces deux matrices ont été immergés dans l'eau pendant 5 min. Des photos de chacune des matrices prises avant et après immersion sont données à la figure 5 annexée.

On peut observer que la matrice M40/60 L⁺ ayant subi l'étape intermédiaire 3bis de lyophilisation du procédé conforme à l'invention gonfle plus vite dans l'eau et atteint son niveau de gonflement définitif (état d'hydratation maximal) en moins de 5 min alors que la matrice M40/60 L⁻ n'ayant pas subi ladite étape gonfle plus lentement et n'atteint pas son niveau d'hydratation maximal dans ce délai. Par conséquent, ces résultats montrent que bien qu'optionnelle, cette étape intermédiaire de lyophilisation permet d'améliorer les propriétés de réhydratation des matrices conformes à l'invention.

## EXEMPLE 3

### Evaluation des effets angiogéniques d'une matrice conforme à l'invention comparativement à une matrice non conforme à la présente invention.

Dans cet exemple, on a évalué les effets angiogéniques de la matrice M40/60 L⁺ conforme à l'invention et telle que préparée ci-dessus à l'exemple 1, comparativement à ceux d'une matrice non conforme à l'invention, obtenue selon un procédé de préparation en tous points identique à celui de la matrice M40/60L⁺ sauf que les deux dernières étapes de déshydratation et de séchage au $CO_2$ supercritique ont été remplacées par une nouvelle étape de congélation puis une nouvelle étape de lyophilisation, lesdites étapes étant effectuées dans les mêmes conditions que les étapes de congélation de la mousse et de lyophilisation de la mousse congelée décrites ci-dessus à l'exemple 1. Ladite matrice, doublement lyophilisée donc, est dénommée M40/60 REF.

Chacune de ces deux matrices a été testée après implantation en l'état chez le rat (matrice M40/60 L⁺ conforme à l'invention et matrice M40/60 REF non conforme à l'invention), ainsi qu'après ensemencement préalable par 500 000 CSMr (matrice M40/60 L⁺-CSM conforme à l'invention et matrice M40/60 REF-CSM non conforme à l'invention). Chaque matrice a été testée sur 2 rats.

Les évaluations ont été effectuées après 28 jours d'implantation.

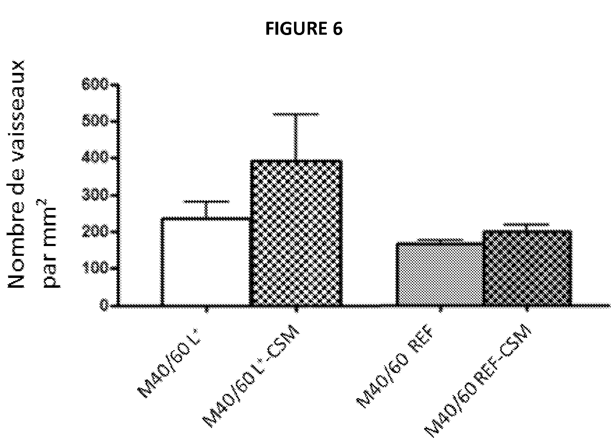

La figure 6 annexée donne le nombre de vaisseaux positifs au $\alpha$-SMA par $mm^2$ après 28 jours d'implantation pour chacune des matrices testées.

Des observations au microscope à fluorescence (non représentées) ont montré que la matrice M40/60 L⁺ implantée est richement vascularisée et que les vaisseaux sont répartis dans la totalité du tissu granulaire qui se forme suite à sa biodégradation. Les vaisseaux présents dans les matrices sont fonctionnels (car ils contiennent des globules rouges). En présence des CSMr ensemencées dans la matrice, la vascularisation de cette dernière est plus importante ($237\pm44$ vaisseaux par $mm^2$ dans le groupe M40/60 L⁺ *versus* $391\pm127$ vaisseaux par $mm^2$ dans le groupe 40/60 L⁺-CSM). Dans le cas de la matrice M40/60 REF non conforme à l'invention, la même tendance est observée en présence de CSM ($166\pm11$ vaisseaux par $mm^2$ *versus* $199\pm21$ vaisseaux par $mm^2$). On constate cependant que la vascularisation de la matrice M40/60 REF est significativement moins abondante que celle de la matrice M40/60 L⁺ séchée au $CO_2$ supercritique conforme à la présente invention.

Ces essais démontrent que le choix de l'étape de séchage final au $CO_2$ supercritique n'est pas une simple alternative à une étape de lyophilisation mais qu'au contraire ce mode de séchage influence les propriétés de la matrice résultante, notamment ses propriétés angiogéniques après implantation.

## Revendications

1. Procédé de préparation d'une matrice polymère biocompatible et biodégradable comportant un réseau de pores ouverts et interconnectés, ledit procédé comportant au moins les étapes suivantes :

   1) la préparation d'une solution aqueuse comprenant au moins un polysaccharide anionique biocompatible et au moins un polymère cationique biocompatible,
   2) l'agitation mécanique de ladite solution obtenue ci-dessus à l'étape précédente en présence d'un agent moussant ou d'un gaz sous pression, pour former une mousse,
   3) la congélation de la mousse obtenue ci-dessus à l'étape précédente, pour obtenir une mousse congelée,
   4) la gélification de la mousse congelée obtenue ci-dessus à l'étape précédente, par ajout à ladite mousse d'au moins un agent gélifiant en solution dans un solvant, pour obtenir une mousse gélifiée, la mousse congelée obtenue ci-dessus à l'étape précédente étant optionnellement lyophilisée,
   5) la déshydratation de la mousse gélifiée obtenue ci-dessus à l'étape précédente, pour obtenir une mousse gélifiée déshydratée, puis
   6) le séchage de la mousse gélifiée déshydratée obtenue ci-dessus à l'étape précédente, par traitement au $CO_2$ supercritique, pour obtenir ladite matrice polymère.

2. Procédé selon la revendication 1, **caractérisé en ce que** le ou les polysaccharides anioniques biocompatibles sont choisis parmi les alginates et alginates modifiés, les pectines, les dérivés cellulosiques, les gommes polysaccharidiques telles que l'agar-agar, la gomme xanthane et la gomme gellane, les carraghénanes, les dextranes modifiés et l'acide hyaluronique, de préférence parmi les alginates et les alginates modifiés.

3. Procédé selon la revendication 1 ou 2, **caractérisé en ce que** la quantité de polysaccharides anioniques présents au sein de la solution aqueuse de l'étape 1) varie de 0,5 à 8 % en masse par rapport à la masse totale de ladite solution aqueuse.

4. Procédé selon l'une quelconque des revendications précédentes, **caractérisé en ce que** les polymères cationiques biocompatibles sont choisis parmi les polysaccharides cationiques, en particulier dans le groupe comprenant le chitosan, des formes salines particulières du chitosan et les dérivés du chitosan ; et les polymères ayant des groupes réactifs basiques tels que des groupes amine ou imine parmi lesquels on peut citer les polyéthylèneimines, les poly(L-lysines), les poly(vinylamines), les poly(aminoacides) et les poly(alkylamines), de préférence parmi le chitosan, des formes salines particulières du chitosan et les dérivés du chitosan.

5. Procédé selon l'une quelconque des revendications précédentes, **caractérisé en ce que** la quantité de polymères cationiques présents au sein de la solution aqueuse de l'étape 1) varie de 0,5 à 15 % en masse par rapport à la masse totale de ladite solution aqueuse.

6. Procédé selon l'une quelconque des revendications précédentes, **caractérisé en ce que** le rapport en masse polysaccharides anioniques ($M_{PA}$) / polymères cationiques ($M_{PC}$) présents dans la solution aqueuse de l'étape 1) varie de 20/80 à 80/20.

7. Procédé selon l'une quelconque des revendications précédentes, **caractérisé en ce que** la solution aqueuse préparée lors de l'étape 1) comprend en outre au moins un tensioactif hydrophile.

8. Procédé selon la revendication 7, **caractérisé en ce que** le ou les tensioactifs hydrophiles représentent de 0,01 à 5 % en masse par rapport à la masse totale de la solution aqueuse de l'étape 1).

9. Procédé selon l'une quelconque des revendications précédentes, **caractérisé en ce que** la mousse est formée en présence d'un agent moussant qui est alors ajouté à la solution préparée à l'étape 1) juste avant la réalisation de l'étape 2).

10. Procédé selon l'une quelconque des revendications 1 à 8, **caractérisé en ce que** lors de l'étape 2), la mousse est formée par introduction d'un gaz sous pression dans la solution aqueuse préparée à l'étape 1).

11. Procédé selon l'une quelconque des revendications précédentes, **caractérisé en ce que** l'étape 3) de congélation est suivie d'une étape 3bis) de lyophilisation de la mousse obtenue à l'issue de l'étape 3).

12. Procédé selon l'une quelconque des revendications précédentes, **caractérisé en ce que** l'agent gélifiant utilisé lors de l'étape 4) de gélification de la mousse congelée est une solution d'au moins un sel d'un cation divalent ou trivalent dans un solvant.

13. Procédé selon l'une quelconque des revendications précédentes, **caractérisé en ce que** l'étape 6) de séchage au $CO_2$ supercritique de la mousse déshydratée obtenue à l'issue de l'étape 5) est réalisé à une température allant de 35 à 50°C, et à une pression allant de 45 à 95 bars.

**Patentansprüche**

1. Verfahren zur Herstellung einer biokompatiblen und biologisch abbaubaren Polymermatrix, die ein Netz aus offenen und miteinander verbundenen Poren aufweist, wobei das Verfahren mindestens folgende Schritte umfasst:

1) Herstellen einer wässrigen Lösung, die mindestens ein biokompatibles anionisches Polysaccharid und mindestens ein biokompatibles kationisches Polymer umfasst,
2) mechanisches Rühren der im vorangehenden Schritt erhaltenen Lösung in Gegenwart eines Schaummittels oder eines Druckgases zum Ausbilden eines Schaums,
3) Gefrierenlassen des im vorangehenden Schritt erhaltenen Schaums zum Erhalten eines gefrorenen Schaums,
4) Gelierenlassen des im vorangehenden Schritt erhaltenen gefrorenen Schaums durch Zugeben von mindestens einem Geliermittel, das sich in einem Lösungsmittel in Lösung befindet, zu dem Schaum zum Erhalten eines gelierten Schaums, wobei der im vorangehenden Schritt erhaltene gefrorene Schaum optional gefriergetrocknet wird,

5) Dehydratisieren des im vorangehenden Schritt erhaltenen gelierten Schaums zum Erhalten eines dehydratisierten gelierten Schaums, dann

6) Trocknen des im vorangehenden Schritt erhaltenen dehydratisierten gelierten Schaums mittels Behandlung mit überkritischem $CO_2$ zum Erhalten der Polymermatrix.

**2.** Verfahren nach Anspruch 1, **dadurch gekennzeichnet, dass** das oder die biokompatiblen anionischen Polysaccharide ausgewählt sind aus Alginaten und modifizierten Alginaten, Pektinen, Cellulosederivaten, Polysaccharid-Gummis wie Agar-Agar, Xanthan und Gellan, Carragenen, modifizierten Dextranen und Hyaluronsäure, vorzugsweise aus Alginaten und modifizierten Alginaten.

**3.** Verfahren nach Anspruch 1 oder 2, **dadurch gekennzeichnet, dass** die Menge an anionischen Polysacchariden, die in der wässrigen Lösung aus Schritt 1) vorhanden sind, von 0,5 bis 8 Gewichts-% bezogen auf das Gesamtgewicht der wässrigen Lösung beträgt.

**4.** Verfahren nach einem der vorhergehenden Ansprüche, **dadurch gekennzeichnet, dass** die biokompatiblen kationischen Polymere aus kationischen Polysacchariden ausgewählt sind, insbesondere aus der Gruppe, die Chitosan, bestimmte Salze von Chitosan und ChitosanDerivate umfasst; sowie aus Polymeren mit reaktionsfähigen basischen Gruppen wie Amin- oder Imingruppen, darunter beispielsweise Polyethylenimine, Poly(L-Lysine), Polyvinylamine, Polyaminosäuren und Polyalkylamine, vorzugsweise aus Chitosan, bestimmten Salzen von Chitosan und Chitosan-Derivaten.

**5.** Verfahren nach einem der vorhergehenden Ansprüche, **dadurch gekennzeichnet, dass** die Menge an kationischen Polymeren, die in der wässrigen Lösung aus Schritt 1) vorhanden sind, von 0,5 bis 15 Gewichts-% bezogen auf das Gesamtgewicht der wässrigen Lösung beträgt.

**6.** Verfahren nach einem der vorhergehenden Ansprüche, **dadurch gekennzeichnet, dass** das Gewichtsverhältnis anionische Polysaccharide ($M_{PA}$) / kationische Polymere ($M_{PC}$) in der wässrigen Lösung aus Schritt 1) von 20/80 bis 80/20 beträgt.

**7.** Verfahren nach einem der vorhergehenden Ansprüche, **dadurch gekennzeichnet, dass** die in Schritt 1) hergestellte wässrige Lösung ferner mindestens ein hydrophiles Tensid umfasst.

**8.** Verfahren nach Anspruch 7, **dadurch gekennzeichnet, dass** das oder die hydrophilen Tenside von 0,01 bis 5 Gewichts-% bezogen auf das Gesamtgewicht der wässrigen Lösung aus Schritt 1) ausmachen.

**9.** Verfahren nach einem der vorhergehenden Ansprüche, **dadurch gekennzeichnet, dass** der Schaum in Gegenwart eines Schaummittels ausgebildet wird, das der in Schritt 1) hergestellten Lösung direkt vor der Durchführung von Schritt 2) zugegeben wird.

**10.** Verfahren nach einem der Ansprüche 1 bis 8, **dadurch gekennzeichnet, dass** in Schritt 2) der Schaum durch Einleiten eines Druckgases in die in Schritt 1) hergestellte Lösung ausgebildet wird.

**11.** Verfahren nach einem der vorhergehenden Ansprüche, **dadurch gekennzeichnet, dass** auf Schritt 3) zum Gefrierenlassen ein Schritt 3a) zum Gefriertrocknen des am Ende von Schritt 3) erhaltenen Schaums folgt.

**12.** Verfahren nach einem der vorhergehenden Ansprüche, **dadurch gekennzeichnet, dass** das Geliermittel, das in Schritt 4) zum Gelieren des gefrorenen Schaums verwendet wird, eine Lösung aus mindestens einem Salz eines zweiwertigen oder dreiwertigen Kations in einem Lösungsmittel ist.

**13.** Verfahren nach einem der vorhergehenden Ansprüche, **dadurch gekennzeichnet, dass** Schritt 6) zum Trocknen des dehydratisierten Schaums, der am Ende von Schritt 5) erhalten wird, mit überkritischem $CO_2$ bei einer Temperatur von 35 bis 50°C und bei einem Druck von 45 bis 95 bar durchgeführt wird.

**Claims**

1. Process for the preparation of a biocompatible and biodegradable polymer matrix comprising a network of open and interconnected pores, said process comprising at least the following stages:

   1) the preparation of an aqueous solution comprising at least one biocompatible anionic polysaccharide and at least one biocompatible cationic polymer,
   2) the mechanical stirring of said solution obtained above in the preceding stage in the presence of a foaming agent or of a pressurized gas, in order to form a foam,
   3) the freezing of the foam obtained above in the preceding stage, in order to obtain a frozen foam,
   4) the gelling of the frozen foam obtained above in the preceding stage, by addition to said foam of at least one gelling agent in solution in a solvent, in order to obtain a gelled foam, the frozen foam obtained above in the preceding stage being optionally lyophilized,
   5) the dehydration of the gelled foam obtained above in the preceding stage, in order to obtain a dehydrated gelled foam, then
   6) the drying of the dehydrated gelled foam obtained above in the preceding stage, by treatment with supercritical $CO_2$, in order to obtain said polymer matrix.

2. Process according to Claim 1, **characterized in that** the biocompatible anionic polysaccharide(s) is (are) chosen from alginates and modified alginates, pectins, cellulose derivatives, polysaccharide gums, such as agar, xanthan gum and gellan gum, carrageenans, modified dextrans and hyaluronic acid, preferably from alginates and modified alginates.

3. Process according to Claim 1 or 2, **characterized in that** the amount of anionic polysaccharides present within the aqueous solution of stage 1) varies from 0.5% to 8% by weight, with respect to the total weight of said aqueous solution.

4. Process according to any one of the preceding claims, **characterized in that** the biocompatible cationic polymers are chosen from cationic polysaccharides, in particular in the group comprising chitosan, particular salt forms of chitosan, and chitosan derivatives; and polymers having basic reactive groups, such as amine or imine groups, among which may be mentioned polyethyleneimines, poly(L-lysines), poly (vinylamines), poly(amino acids) and poly(alkylamines), preferably from chitosan, specific salt forms of chitosan, and chitosan derivatives.

5. Process according to any one of the preceding claims, **characterized in that** the amount of cationic polymers present within the aqueous solution of stage 1) varies from 0.5% to 15% by weight, with respect to the total weight of said aqueous solution.

6. Process according to any one of the preceding claims, **characterized in that** the ratio by weight anionic polysaccharides ($W_{AP}$)/cationic polymers ($W_{CP}$) which are present in the aqueous solution of stage 1) varies from 20/80 to 80/20.

7. Process according to any one of the preceding claims, **characterized in that** the aqueous solution prepared during stage 1) additionally comprises at least one hydrophilic surfactant.

8. Process according to Claim 7, **characterized in that** the hydrophilic surfactant(s) represent(s) from 0.01% to 5% by weight, with respect to the total weight of the aqueous solution of stage 1).

9. Process according to any one of the preceding claims, **characterized in that** the foam is formed in the presence of a foaming agent which is then added to the solution prepared in stage 1) just before carrying out stage 2).

10. Process according to any one of Claims 1 to 8, **characterized in that**, in stage 2), the foam is formed by introduction of a pressurized gas into the aqueous solution prepared in stage 1).

11. Process according to any one of the preceding claims, **characterized in that** the freezing stage 3) is followed by a stage 3a) of lyophilization of the foam obtained on conclusion of stage 3).

12. Process according to any one of the preceding claims, **characterized in that** the gelling agent used during stage 4) of gelling the frozen foam is a solution of at least one salt of a divalent or trivalent cation in a solvent.

13. Process according to any one of the preceding claims, **characterized in that** stage 6) of drying, with supercritical $CO_2$, the dehydrated foam obtained on conclusion of stage 5) is carried out at a temperature ranging from 35°C to 50°C and at a pressure ranging from 45 to 95 bar.

**FIGURE 1**

## FIGURE 2

a

b

## FIGURE 3

## FIGURE 4

## FIGURE 5

# FIGURE 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007103208 A

- US 6425918 B

**Littérature non-brevet citée dans la description**

- **ANDERSEN T. et al.** *BioMacromolecules*, 2012, vol. 13, 3703-3710
- **CHHAVI SHARMA et al.** *Journal of Applied Polymer Science*, 2012, vol. 127 (4), 3228-3241

- **SHAPIRO L et al.** *Biomaterials*, 1997, vol. 18, 583-590
- **ANDERSEN et al.** *Biomacromolecules*, 2012
- **KONG H.J. et al.** *Polymer*, 2002, vol. 43, 6239-6246